# EUROPEAN PATENT APPLICATION

(11) **EP 4 016 491 A1**
(43) Date of publication of application: **22.06.2022**
(21) Application number: 20214426.7
(22) Date of filing: 16.12.2020
(51) Int. Cl.: G08C 17/02, A61B 17/00

(54) **COMMUNICATIONS SYSTEM FOR A SURGICAL DEVICE, SURGICAL SYSTEM, AND METHOD OF CONTROL**

(71) Applicant: Leica Instruments (Singapore) Pte. Ltd., 608924 Singapore (SG)
(72) Inventor: NITIN, Roy, 560102 Bangalore (IN)
(74) Representative: 2SPL Patentanwälte PartG mbB

(57) **Abstract**

A communications system for a surgical device which includes a broker engine (BE) is disclosed. The BE has a serial communications interface which communicatively couples to a surgical device, and a wireless communications interface which communicatively couples to a remote device. The wireless communications interface receives an incoming instruction from the remote device. The BE performs tasks such as transmitting a control instruction based on the incoming instruction, the transmission being via the serial communications interface. The control instructions control the surgical device.

## Description

### Technical field

Examples disclosed herein relate to systems for surgical devices such as microscopes.

### Background

Modern surgical devices may have numerous functions. For example, surgical microscopes may allow a user to visualize anatomy at fine resolution. Surgical devices such as microscopes may be precisely controlled. It can be challenging to improve user experiences with medical devices, such as surgical microscopes, for example due to the extreme precision in movement that may be required.

A surgical suite where a surgical device may be found may be a highly controlled environment subject to frequent deep cleaning. Surgical devices may also collect/store data which can be transferred to medical personnel outside of the surgical suite.

Surgical device user interfaces are often keyboards, touchscreens, switches, and the like, which may use a form of physical contact for their function.

### Summary

It may be desirable to remotely use/access surgical devices, and to reduce the amount of physical contact of medical personal with surgical devices. It may be desirable to have a means of controlling a surgical device which may reduce the frequency and/or extent of physical contact between medical personnel and surgical devices for reduced transmission/contamination of microbes. Alternatively/additionally, less cabling can be made possible with the disclosed devices, methods, and systems. Less cabling is particularly desirable in surgical suites, providing more freedom of movement. Devices, systems, and methods of controlling surgical devices are disclosed herein to address such problems.

Herein is disclosed a communications system for a surgical device as defined in claim 1, and a surgical device as defined in claim 13. Herein is disclosed a method of controlling a surgical device as defined in claim 14, and a computer program and defined in claim 15.

In an embodiment, a communications system for a surgical device includes a broker engine (BE). The BE has a serial communications interface which communicatively couples to a surgical device, and a wireless communications interface which communicatively couples to a remote device. The wireless communications interface may receive an incoming instruction from the remote device. The BE may perform tasks such as transmitting a control instruction based on the incoming instruction, the transmission being via the serial communications interface. The control instruction may control the surgical device. The communications system may thereby reduce physical contact between the user and the surgical device, *e.g.* by having a control means which is remote from the surgical device.

In an embodiment, the wireless communications interface is for hosting event based bidirectional communication, and the wireless communications interface is for communicatively coupling via the event based bidirectional communication to the remote device. The wireless communications interface is for receiving the incoming instruction via the event based bidirectional communication. Two-way communication may be enabled by event based bidirectional communication.

In an embodiment, the tasks may include, for example, transmitting data received by the BE broker through the serial communications interface from the surgical device via the wireless communications interface. The communication system can thereby provide the data to the remote device. It is desirable for the remote device to receive data originating at the surgical device.

In an embodiment, the broker engine is configured to transmit a homepage via the event based bidirectional communication for receipt by the remote device. The homepage can be based on a memory homepage stored in a memory of the broker engine. A user, on the remote device, can thereby see the homepage and, for example, the connection can be initiated and/or confirmed.

In an embodiment, the control instruction includes at least one of: an initiate command to initiate a software update of the surgical device; an adjust command to adjust a setting of the surgical device; a setting return command to return a setting of the surgical device; and a parameter return command to return a parameter of the surgical device. It is advantageous for a user to be able to remotely control the surgical device in such a manner. The surgical device's availability is improved.

In an embodiment, the plurality of tasks includes at least one of: determine a completion of a software update of the surgical device; compare a parameter or a setting with a check-value; perform a diagnostic on the surgical device; generate a diagnostic report of the surgical device; initiate capturing of data, initiate exporting data, initiate a diagnostic test of a motor, enable/disable a working distance setting, initiate a zoom, initiate a diagnostic of zoom, and initiate a motor motion. It is particularly advantageous for a user to be able to remotely control the tasks as listed of the surgical device in such a manner. The surgical device's availability is improved.

In an embodiment, the parameter is at least one of: a feedback parameter such as a task completion, a used operation time of a lamp, a test state, a limit of motor position, a range of motor, a light source information, a light meter state, a light meter value, an observation filter set state, an optical carrier voltage, and optics carrier configuration, a state of movement of a motor, a state of a switch. It is advantageous for a user to be able to remotely determine parameters such as those listed above. The surgical device's availability is improved.

In an embodiment, the setting is at least one of: a magnification, a lens selection, a zoom, a focus, a brightness, a voltage, a shutter state, a motor position, a motor speed, a fluorescence mode, a filter wheel state, a switch selection, a footswitch assignment, a key assignment, a display configuration, a motor lock, and a focus lock. It is advantageous for a user to be able to remotely adjust, set, determine, and/or compare settings such as those listed above. The surgical device's availability is improved.

In an embodiment, the diagnostic is at least one of: determining if a light source is operational, determining if a motor is operational, and determining if a feedback signal is operational. Remote diagnostics can reduce the operation costs, for example by allowing remote diagnostics rather than hands-on diagnostics.

In an embodiment, the BE allows the remote device to connect via the event based bidirectional communication after a successful authentication; and the BE blocks a remote device from connecting to the event based bidirectional communication after an unsuccessful authentication. Authentication of users can enhance the security of the surgical device.

In an embodiment, a successful authentication includes confirmation that a received user data which is received by the broker engine via the event based bidirectional communication matches a stored user data that is stored in a memory 157) of the broker engine. Authentication of users can enhance the security of the surgical device.

In an embodiment, the communications system includes a memory and a processor configured to perform at least one of the plurality of tasks. The wireless communications interface is a TCP connection for hosting an event based bidirectional communication for simultaneously communicatively coupling via event based bidirectional communication to a plurality of remote devices. The event based bidirectional communication can receive a plurality of incoming instructions from the plurality of remote devices. The communications system may thereby reduce physical contact between the user and the surgical device, *e.g.* by having a control means which is remote from the surgical device.

Herein is disclosed, according to an embodiment, a surgical system which includes a surgical device and the communications system as disclosed herein. The surgical system may reduce physical contact between the user and the surgical device, *e.g.* by enabling a control means which is remote from the surgical device.

Herein is disclosed, according to an embodiment, a method of controlling a surgical device, including: providing a wireless communications interface for coupling a remote device to a broker engine which is communicatively coupled to a surgical device through a serial communications interface; receiving an incoming instruction via the wireless communications interface; transmitting a control instruction based on the incoming instruction via the serial communications interface for controlling the surgical device; and controlling a task of the surgical device with the control instructions. Advantages may include and are not limited by making less cabling possible in a surgical suite.

Herein is disclosed, according to an embodiment, a computer program with a program code for performing the methods described herein, on the communications system (100) as described herein, or on the surgical system as described herein.

### Short Description of the Figures

Some examples of apparatuses and/or methods will be described in the following by way of example, and with reference to the accompanying figures, in which
Fig. 1 illustrates a surgical system including a communications system for a surgical device, according to an embodiment described herein;
Fig. 2 illustrates a surgical system including a communications system for a surgical device, according to an embodiment described herein;
Fig. 3 illustrate a method of controlling a surgical device, according to an embodiment described herein;
Fig. 4 illustrates a surgical system including a communications system for a surgical device, according to an embodiment described herein; and
Fig. 5 illustrates a surgical system according to an embodiment described herein.

### Detailed Description

Various examples will now be described more fully with reference to the accompanying drawings in which some examples are illustrated. In the figures, which are not to be assumed to be to scale, the thicknesses of lines, layers and/or regions may be exaggerated for clarity.

As used herein, the term "and/or" includes any and all combinations of one or more of the associated listed items and may be abbreviated as "/". Herein, a trailing "(s)" indicates one or more; for example remote device(s) indicates one or more remote devices. Herein to "retrieve" a setting/parameter may be used interchangeably with to "return" a setting/parameter. Herein, "instrument" may be used interchangeably with "surgical device."

Herein, communications systems are described, which may be illustrated as being in communication with a surgical device, particularly a surgical microscope. The communications systems described herein may be physically/communicatively coupled to other surgical devices. The communications systems described herein may be specifically configured/programmed to operate a particular kind of surgical device such as a surgical microscope. The configured/programmed communications system may be disconnected from a surgical device, *e.g.* physical and communicatively disconnected. A communications system may be operational when, for example, plugged into a serial communications port of a surgical device, such as a surgical microscope, for example. Herein, an event based bidirectional communication may be between a remote device and the broker engine as described herein; a processor may be configured to host event based bidirectional communication as described herein.

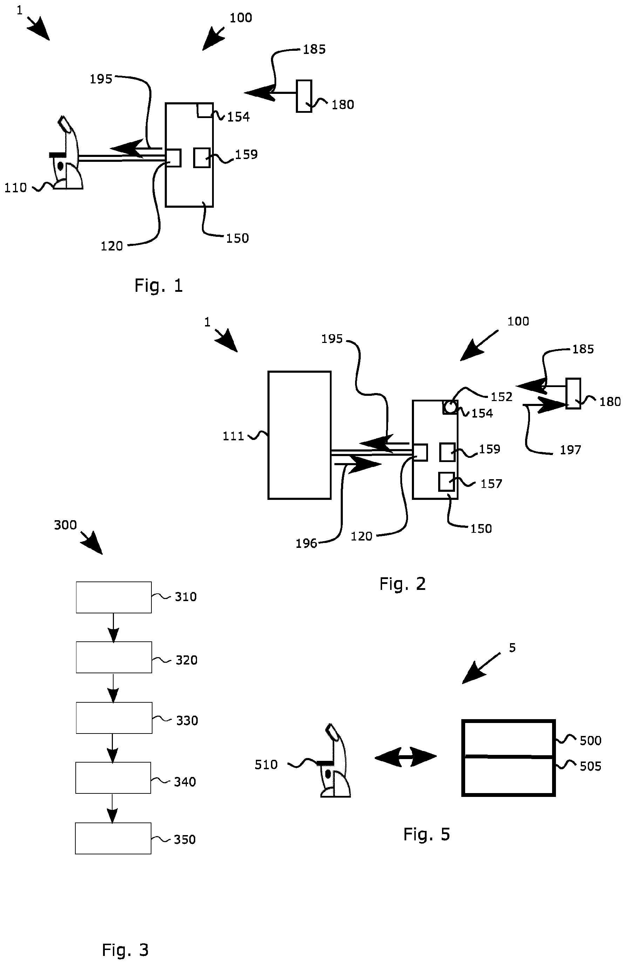

Fig. 1 illustrates an embodiment of a surgical system 1 including communications system 100 for a surgical device 110, such as a microscope. The communications system 100 includes a broker engine (BE) 150. The BE 150 has a serial communications interface 120 which communicatively couples to a surgical device 110, and a wireless communications interface 154 which communicatively couples to a remote device 180. The wireless communications interface 154 may receive an incoming instruction 185 from the remote device 180. The broker engine may be powered through a universal serial bus, which may also act as the medium for data exchange, *e.g.* the serial communications interface 120.

The BE 150 may perform tasks such as transmitting a control instruction 195 based on the incoming instruction 185, the transmission being via the serial communications interface 120. The control instruction 195 may control the surgical device 110. The communications system 100 may thereby reduce physical contact between the user and the surgical device, *e.g.* by having a control means which is remote from the surgical device.

The devices, systems, and methods described herein may reduce the need of a user to be present in front of a surgical device to perform tasks. Diagnostics and initial instrument monitoring can be done remotely. Reducing physical contact may reduce the chance of transmission/contamination of microbes, which is particularly important in surgical suites in which a surgical system 1 and/or surgical device 110 may be found.

The communications system 100 described herein may be useful for any medical device that supports serial bus communication. Herein, there is described the use of the communications system 100 with a surgical microscope. The communications system 100 may facilitate preparation of surgical devices, such as microscopes, for surgery, *e.g.* adjustable functionalities like auto balance, footswitch assignment, and/or key assignments can be checked/adjusted remotely
Alternatively/additionally, tasks which can be performed by the BE 150 can include at least one of: determine a completion of a software update of the surgical device; compare a parameter or a setting with a check-value; perform a diagnostic on the surgical device; generate a diagnostic report of the surgical device; initiate capturing of data, initiate exporting data, initiate a diagnostic test of a motor, initiate a motor motion, enable/disable a working distance setting, initiate a zoom, and initiate a diagnostic of zoom. It is particularly advantageous for a user to be able to remotely control the tasks as listed of the surgical device in such a manner. The surgical device's availability is improved. For example, diagnostics can be performed remotely and may reduce the cost of ownership by allowing remote service engineers to determine user error off-site. Software upgrades can be performed in a better and more efficient way. It is also handy, and reduces the risk of microbial contamination, for users on-site to be able to export data, *e.g.* to a remote device 180, without physically contacting the surgical device 110 and/or be present in the surgical suite.

A control instruction 195, which is transmitted to the surgical device 110, may include at least one of: an initiate command to initiate a software update of the surgical device; an adjust command to adjust a setting of the surgical device 110; a setting return command to return a setting of the surgical device 110; and a parameter return command to return a parameter of the surgical device 110. It is advantageous for a user to be able to remotely control the surgical device in such a manner. The surgical device's availability may thereby be improved.

Fig. 2 illustrates an embodiment of a surgical system 1 including communications system 100 for a surgical device 110, such as a microscope. The wireless communications interface 154 may be for hosting event based bidirectional communication 152, and the wireless communications interface 154 may communicatively couple via the event based bidirectional communication 152 to one or more remote devices 180. The wireless communications interface 154, which may include a TCP connection, can receive the incoming instruction 185 via the event based bidirectional communication 152. Event based bidirectional communication 152 may be advantageous for providing two-way communication. For example, event based bidirectional communication may be capable of full duplex over a single TCP connection. Event based bidirectional communication may improve user interactions with devices.

The communications system 100 can include a memory 157 and a processor 159 configured to perform at least one of the plurality of tasks. The wireless communications interface 154 may be a TCP connection for hosting event based bidirectional communication 152 for simultaneously communicatively coupling via the event based bidirectional communication 152 to a plurality of remote devices 180. The event based bidirectional communication 152 can receive a plurality of incoming instructions 185 from the plurality of remote devices 180. The communications system 100 may thereby reduce physical contact between the user and the surgical device 110, *e.g.* by having a control means which is remote from the surgical device 110.

The memory 157 may include a nonremovable storage medium and/or a removable storage medium. For example, a removable storage medium may be used to transfer/export data/images.

Fig. 2 illustrates the possible transmission of data 196, 197 which can be received by the BE 159 through the serial communications interface 120 from the surgical device 110 via the wireless communications interface 154, *e.g.* to one or more remote devices 180. Alternative/additional tasks which may be performed by the BE 150 may include transmitting data 196, 197 received by the broker engine 150 through the serial communications interface 120 from the surgical device 110 via the wireless communications interface 154. The BE 150 may provide the data 196 to the remote device 180. The data 196 can be transmitted as data 197 to the remote device(s) 180. For example, data 196 can be processed by the communications system 100, and forwarded to the remote device(s) 180 as processed data 197. Alternatively/additionally, the data 196 received by the BE 150 can be stored in memory 157 of the communications system 100, *e.g.* for later analysis/transmission, as received and/or as processed.

The broker engine 150 may be configured to transmit a homepage via the event based bidirectional communication 152 for receipt by the remote device(s) 180. The homepage can be based on a memory homepage stored in the memory 157 of the broker engine 150. A user, on a remote device 180, can thereby see the homepage. A user may initiate and/or confirm the communication connection between the remote device 180 and the communications system 100.

The broker engine 150 may provide a wi-fi capability to the surgical device 110. For example, the broker engine can be configured as a soft access point which can enable the module to host its own wi-fi network with an option to introduce a proper authentication.

The BE 150, acting as a server, can exchange data between handheld devices like smartphones, tablets, and the like via TCP, particularly event based bidirectional communication, which may operate full-duplex, bi-directional and at single TCP connection. The BE 150 may forward requests from the remote device(s)s 180 to the surgical instrument 110 over the serial communications interface 120.

Upon successful creation of an event based bidirectional communication connection, a remote device 180 can join the network hosted by the broker engine 150, and register itself as a client only after a successful authentication. After registration, the remote device 180 can send a request for a webpage. The broker engine 150 services the request by sending a homepage(s) located in memory. For example, the registration may ensure that the remote device 180 is properly connected with the server over the hosted event based bidirectional communication, such that the remote device 180 can send any request which will be received by the broker engine 150.

If the request sent is to execute some commands in the surgical device 110, the broker engine 150 also may have the protocol implemented which converts the requests from remote devices 180, to messages as per possibly defined protocol communication for serial exchange of data. Post conversion, the commands can be executed, and results sent back to the webserver on the broker engine 150 via the serial communication 120.

The BE 150 may, as described herein, transmit control instructions 195 which are a kind ofa parameter return command to return a parameter of the surgical device 110. There may be a plurality of parameters which each may be returned, depending on the specifics of the parameter return command.

Examples of parameters which may be returned, determined, set, or changed, or the like, are at least one of: a feedback parameter (which includes a notice of a completion of a task), a used operation time of a lamp (which may indicate the number of hours a lamp has been used), a test state (which may indicate that a test is in progress, optionally with an indication of time elapsed/remaining), a limit of motor position, a range of motor, a light source information (such as kind, *e.g.* xenon lamp, LED, tungsten filament, *etc.*)*,* a light meter state, a light meter value, an observation filter set state, an optical carrier voltage, and optics carrier configuration, a state of movement of a motor, a state of a switch. It is advantageous for a user to be able to remotely determine parameters such as those listed above. The surgical device's availability is improved. For example, it may be useful for maintenance purposes to be able to remotely determine such parameters. It is particularly advantageous to be able to do so with a reduced risk of microbial contamination of the surgical environment/device.

The same graphical user interface, GUI, can be used to login to multiple instruments within a network and can be controlled independently. Some tasks can be initiated for multiple instruments at a time and can be monitored. The GUI can be small and located closer to the user/surgeon. It can be possible to perform remote diagnostics and get reports out of the instrument on the fly. Settings/parameters can be ported from one microscope to another, thus ensuring faster tuning of the parameters. Comparisons of the parameters across the microscopes can be made more easily.

The BE 150 may, as described herein, transmit a control instruction 195 which is a setting return command to return a setting of the surgical device 110. The BE 150 may also initiate an adjustment of a setting of the surgical device 110. There may be a plurality of settings which each may be returned, depending on the specifics of the setting return command.

Examples of settings which may be returned, determined, adjusted, set, or changed, or the like, are at least one of: a magnification, a lens selection, a zoom, a focus, a brightness, a voltage, a shutter state, a motor position, a motor speed, a fluorescence mode, a filter wheel state, and a switch selection, a footswitch assignment, a key assignment, a display configuration, a motor lock, a focus lock. It is advantageous for a user to be able to remotely adjust, set, determine, and/or compare settings such as those listed above. The surgical device's availability is improved.

As described herein, the tasks which the BE 150 may be configured to perform may also include diagnostics, *e.g.* the performance of a diagnostic and/or generation of a diagnostic report. There may be one or more diagnostics such as at least one of: determining if a light source is operational, determining if a motor is operational, and determining if a feedback signal is operational. Remote diagnostics can reduce the operation costs, for example by allowing remote diagnostics alternatively to or in addition to hands-on diagnostics.

The BE 150 may allow the remote device 180 to connect via the event based bidirectional communication 152 after a successful authentication. The BE 150 may block a remote device 180 from connecting to the event based bidirectional communication 152 after an unsuccessful authentication. Such authentication of users can enhance the security of the surgical device 110 and/or any stored data. A successful authentication may include confirmation that a received user data which is received by the broker engine 150 via the event based bidirectional communication 152 matches a stored user data that is stored in a memory 157 of the broker engine 150.

As described herein, a surgical system 1 can includes a surgical device 110, such as a surgical microscope, and the communications system 100 as described herein in its various embodiments. The surgical system may reduce physical contact between the user and the surgical device, *e.g.* by enabling a control means which is remote from the surgical device.

Fig. 3 illustrates, according to an embodiment, a method 300 of controlling a surgical device. The method 300 includes communicatively coupling 310 a surgical device through a serial communications interface 120) to a broker engine. A remote device is coupled 320 with a wireless communications interface of the broker engine. An incoming instruction is received 330 via the wireless communications interface. A control instruction is transmitted 340 based on the incoming instruction via the serial communications interface for controlling the surgical device. A task of the surgical device is controlled 350 with the control instructions. The method illustrated in Fig. 3 can include the functionalities described herein for the surgical systems 1, surgical devices 110 (such as microscopes), and/or communications systems 100.

According to an embodiment, a computer program has code for performing the method(s) 300 described herein, on the communications system 100 as described herein, and/or on the surgical system 1 as described herein.

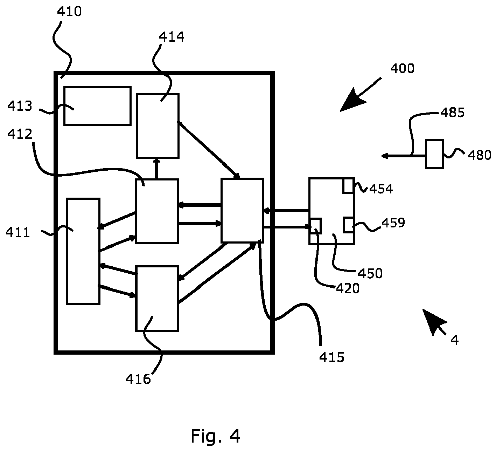

Fig. 4 illustrates, according to an embodiment, a block diagram of a surgical system 4 including communications system 400 for a surgical device 410, such as a microscope. The surgical system 4 represented in Fig. 4 can be compared to that of Figs. 1 and 2, as well as the method illustrated in Fig. 3. The communications system 400 of Fig. is comparable with that described in relation to Figs. 1, 2, and 3, and may be separated from the surgical device 410.

The surgical systems 1, 4 described herein optionally include at least one remote device 180, 480.

The communications system 400 depicted in Fig. 4 includes a broker engine (BE) 450. The BE 450 has a serial communications interface 420 which communicatively couples to a surgical device 410, and a wireless communications interface 454 which communicatively couples to a remote device 480. The wireless communications interface 454 may receive an incoming instruction 485 from the remote device 480. The communications system 400 can include a processor 459 configured to perform at least one of the plurality of tasks as described herein.

Fig. 4 illustrates several optional functionalities particularly of the surgical device 410 which may include memory storage 411. The surgical device 410 can include a serial communications port 415 which is in communication with that of the BE 450. Access to memory 411 may depend on user characteristics. Memory elements of the memory storage 411 can be read/written, as indicated by the arrows connecting the memory storage 411 to the blocks representing user configuration 412 and system configuration 416. Some settings and/or parameters, which are writable (*e.g.* can be set and/or changed) through the system configuration block 416, can be changed by some users, *e.g.* service personnel, and may not be writable (changeable) for other users, *e.g.* standard users, *e.g.* when access is through the user configuration 412 block.

Identification of a user's permissions may be done by the BE 150. The user's permissions may determine whether the user may access parameters through the system configuration block 416 and/or the user configuration block 412.

Preferred profiles (*e.g.* user parameters/settings) can be loaded remotely. A user may import user preferences (*e.g.* parameters/settings) from one similar device to another.

For users such as surgeons, it may be possible to manage accounts, *e.g.* create accounts and login. Settings/parameters can be reviewed and ported from one instrument to another similar kind of instrument, based on the user's profile/preferences. Recordings and snapshots can be saved on the surgeon's drive on the fly, *e.g.* on the remote device 480. Mechanisms can be leveraged to control devices like endoscope and image guided surgery.

The memory storage 411 can store possible settings, possible parameters, and menus for selection from possible settings and/or parameters. Various users, *e.g.* those with system configuration access 416 (*e.g.* service personnel) and/or those with user configuration 412 access (*e.g*. other users) may be able to apply possible settings/parameters to the surgical device 410. Parameters/settings may be selected/determined by a user with access through the user configuration block 412 and applied to the actual parameter/setting 414 of the surgical device 410. The underlying device parameter/setting 413 may then be adjusted.

A return command may result in transmission of an actual parameter/setting 414 to the BE 450 and/or through the BE 450 to a remote device 480. Actual parameter/settings 414, such as motor positions and speeds, may be directly changed, for example. The user configuration 412 may alternatively/additionally represent user defined initial values which are applied/copied to actual parameters/settings 414 at startup and/or upon selection which may depend on the identification of the user (*e.g.* the user's permissions which may be stored in memory of the surgical device 410 and/or BE 450).

Users may have stored user configurations 412 which are activated/used upon start-up. Parameters/setting accessible through the system configuration 416 may be system wide values which are, for example, only changed by authorized users such as service personnel. For example, a list of accessories that are installed in the surgical device 410 can be accessible in the system configuration 416, *e.g.* for modification.

Retrieval of data from the surgical device 410 is possible. For example, data 196 can be displayed on a graphical user interface which may be on the surgical instrument 410 and/or remote device(s) 480. With reference also to Fig. 2, the BE 450 may process the data 196 before transmitting the data 197 to the remote device(s) 480, for example. For example, the remote device(s) 480 may display data 197 such as the parameter(s), setting(s), image data, and the like. The display of the remote device(s) 480 may supplement, copy, or replace display information that the surgical device 410 may be capable of displaying. The ability to display such data through the remote device(s) may increase convenience for standard users and/or service personnel. The communications system (100) may have modes which allow for picture-in-picture, side-by-side, and full screen mode display.

The communications system 400 may allow management of user profiles. For example, the communications system 400 may allow a user to select/load various settings based on the user identity. For example, each profile may have information related to magnification, working distance, handle configurations, key assignment, footswitch assignment/configuration, various configurable values in supported operating modes such as brightness values, illumination values, image parameter values like homogenization, color saturation, and intensities of overlays. It may be particularly advantageous to allow remote access via the communications system 100, 400 as described herein to the assignment of keys (such as those on handles and/or joysticks), and footswitches. It is convenient to retrieve/set the basic settings/parameters of the surgical device 410 remotely via the communications systems 100, 400 as described herein, *e.g.* through a remote device 180, 480.

The communications system 400 may alternatively/additionally have mechanisms/methods to (i) initiate capturing of data, for example in the form of screenshots, (ii) initiate commands to capture recordings, (iii) initiate commands to export the data, *e.g.* to a connected media drive. A user may be able to view or select among captured screenshots and/or recordings and export the same. Such mechanisms may be further optional tasks which the broker engine 450 is configured to perform, and/or further features of methods described herein.

Some embodiments relate to a surgical system 1, 4 which may include a microscope as described in connection with one or more of the Figs. 1-4. Additionally/alternatively, a microscope may be part of or connected to a system as described in connection with one or more of the Figs. 1-4.

Fig. 5 shows a schematic illustration of a surgical system 5 configured to perform a method described herein. The system 5 comprises a microscope 510 and a computer system 505. The microscope 510 is configured to take images and is connected to the computer system 505. The computer system 505 is configured to execute at least a part of a method described herein. The computer system 505 may be configured to execute a machine learning algorithm. The computer system 505 and microscope 510 may be separate entities but can also be integrated together in one common housing. The computer system 505 may be part of a central processing system of the microscope 510 and/or the computer system 505 may be part of a subcomponent of the microscope 510, such as a sensor, an actor, a camera or an illumination unit, etc. of the microscope 510.

The computer system 505 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 505 may comprise any circuit or combination of circuits.

The computer system 505 may include a communications system 500 as described herein, such as a removable communications system 500 connected to a serial communications port.

In one embodiment, the computer system 505 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 505 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 505 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 505 may also include a display device, one or more speakers, and a keyboard and/or controller, which can include a mouse, trackball, touch screen, voice-recognition device, or any other device that permits a system user to input information into and receive information from the computer system 505.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

**List of reference signs**

| | |
|---|---|
| surgical system | 1, 4, 5 |
| communications system | 100, 400, 500 |
| surgical device | 110,410 |
| serial comm. interface | 120, 420 |
| | |
| broker | 150, 450 |
| engine event based bidirectional communication | 152, 452 |
| wireless communications interface | 154, 454 |
| memory | 157 |
| processor | 159, 459 |
| | |
| remote device(s) | 180,480 |
| incoming instructions | 185, 485 |
| control instructions | 195 |
| | |
| data | 196 |
| data | 197 |
| | |
| method of controlling a surgical device | 300 |
| communicatively coupling | 310 |
| coupling | 320 |
| receiving | 330 |
| controlling | 340 |
| | |
| computer system | 505 |
| microscope | 510 |

## Claims

1. A communications system (100) for a surgical device (110), comprising:
a broker engine (150) comprising
a serial communications interface (120) for communicatively coupling to a surgical device (110); and
a wireless communications interface (154) for communicatively coupling to a remote device (180), the wireless communications interface (154) for receiving an incoming instruction (185) from the remote device (180); wherein
the broker engine (150) is configured to perform a plurality of tasks comprising:
transmitting a control instruction (195) based on the incoming instruction (185) via the serial communications interface for controlling the surgical device (110).

2. The communications system (100) of any preceding claim, wherein
the wireless communications interface (120) is for hosting event based bidirectional communication; and the wireless communications interface (120) is for communicatively coupling via the event based bidirectional communication to the remote device (180); and the wireless communications interface (120) is for receiving the incoming instruction (185) via the event based bidirectional communication.

3. The communications system (100) of any preceding claim, wherein
the plurality of tasks further comprises:
transmitting data (196) received by the broker engine (150) through the serial communications interface (120) from the surgical device (110) via the wireless communications interface (154) for providing the data (196) to the remote device (180).

4. The communications system (100) of any of claims 2-3, wherein
the broker engine (150) is configured to transmit a homepage via the event based bidirectional communication for receipt by the remote device (180); wherein
the homepage is based on a memory homepage stored in a memory (157) of the broker engine (150).

5. The communications system (100) of any preceding claim, wherein
the control instruction (195) comprises at least one of:
an initiate command to initiate a software update of the surgical device (110);
an adjust command to adjust a setting of the surgical device (110);
a setting return command to return a setting of the surgical device (110); and
a parameter return command to return a parameter of the surgical device (110).

6. The communications system (100) of any preceding claim, wherein
the plurality of tasks further comprises at least one of:
determine a completion of a software update of the surgical device (110);
compare a parameter or a setting with a check-value;
perform a diagnostic on the surgical device (110);
generate a diagnostic report of the surgical device (110);
initiate capturing of data;
initiate exporting data, initiate a diagnostic test of a motor;
enable/disable a working distance setting;
initiate a zoom;
initiate a diagnostic of zoom; and
initiate a motor motion.

7. The communications system (100) of claim 5 or 6, wherein
the parameter is at least one of:
a feedback parameter such as a task completion, a used operation time of a lamp, a test state, a limit of motor position, a range of motor, a light source information, a light meter state, a light meter value, an observation filter set state, an optical carrier voltage, and optics carrier configuration, a state of movement of a motor, a state of a switch.

8. The communications system (100) of any of claims 5-7, wherein
the setting is at least one of: a magnification, a lens selection, a zoom, a focus, a brightness, a voltage, a shutter state, a motor position, a motor speed, a fluorescence mode, a filter wheel state, a switch selection, a footswitch assignment, a key assignment, a display configuration, a motor lock, and a focus lock.

9. The communications system (100) of any of claims 5-8, wherein
the diagnostic is at least one of: determining if a light source is operational, determining if a motor is operational, and determining if a feedback signal is operational.

10. The communications system (100) of any of claims 2-9, wherein
the broker engine is for:
allowing the remote device to connect via the event based bidirectional communication after a successful authentication; and
blocking the remote device from connecting to the event based bidirectional communication after an unsuccessful authentication.

11. The communications system (100) of claim 10, wherein
a successful authentication comprises:
confirmation that a received user data which is received by the broker engine (150) via the event based bidirectional communication matches a stored user data that is stored in a memory (157) of the broker engine (150).

12. The communications system (100) of any of claims 2-11, configured to control a surgical microscope, further comprising
a memory (157) and a processor (159) configured to perform at least one of the plurality of tasks; wherein
the wireless communications interface (154) is a TCP connection configured for hosting an event based bidirectional communication (152) for simultaneously communicatively coupling via the event based bidirectional communication (152) to a plurality of remote devices (180), the event based bidirectional communication (152) configured to receive a plurality of incoming instructions from the plurality of remote devices (180).

13. A surgical system (1) including the communications system (100) of any preceding claim, and including a surgical device (110) which is optionally a surgical microscope, the surgical system (100) further optionally including the remote device (180).

14. A method of controlling a surgical device (110), comprising:
providing a wireless communications interface (120) for coupling a remote device (180) to a broker engine (150) which is communicatively coupled to a surgical device (110) through a serial communications interface (120);
receiving an incoming instruction (185) via the wireless communications interface (120);
transmitting a control instruction (195) based on the incoming instruction (185) via the serial communications interface (120) for controlling the surgical device (110); and
controlling a task of the surgical device (110) with the control instructions (195).

15. A computer program with a program code for:
performing the method according to claim 14 on the communications system (100) of any of claims 1-12, or the surgical system of claim 13.
